# EUROPÄISCHE PATENTSCHRIFT

(11) **EP 1 250 163 B1**
(45) Veröffentlichungstag und Bekanntmachung des Hinweises auf die Patenterteilung: **15.12.2004**
(21) Anmeldenummer: 01915159.6
(22) Anmeldetag: 25.01.2001
(51) Int. Cl.: A61L 27/12, A61L 27/56, A61L 27/42, A61L 27/58

(54) **ANORGANISCHES RESORBIERBARES KNOCHENERSATZMATERIAL UND HERSTELLUNGSVERFAHREN**
INORGANIC RESORBABLE BONE SUBSTITUTE MATERIAL AND PRODUCTION METHOD
MATERIAU OSSEUX DE SUBSTITUTION INORGANIQUE RESORBABLE ET PROCEDE PERMETTANT DE LE PRODUIRE

(30) Priorität: 28.01.2000 DE 10003824; 02.12.2000 DE 10060036
(43) Veröffentlichungstag der Anmeldung: 23.10.2002
(73) Patentinhaber: DOT GmbH, 18059 Rostock (DE)
(72) Erfinder: GERBER, Thomas, 18059 Papendorf (DE)
(74) Vertreter: Schnick, Achim
(86) Internationale Anmeldenummer: PCT/EP2001/000803
(87) Internationale Veröffentlichungsnummer: WO 2001/054747

(56) Entgegenhaltungen:
- WO-A-99/34844
- DE-A- 19 721 661
- DE-A- 19 825 419
- US-A- 5 652 056

## Beschreibung

Die Erfindung betrifft ein Verfahren zur Herstellung eines anorganisches resorbierbares Knochenersatzmaterial auf der Basis von Calciumphosphaten.
Die Transplantation von Knochen ist nach der Applikation von Blutbestandteilen die zweithäufigste Transplantationsform beim Menschen (Fox, R.: New bone The Lancet 339, 463f. (1992)). So wurden in den USA 1993 250000 Knochentransplantationen durchgeführt (Kenley et al.: Biotechnology and bone graft substitutes. Pharmaceut. Res. 10, 1393 (1993)). Der Ersatz posttraumatischer, als Folge von Osteomyelitiden und Tumoroperationen auftretender sowie osteoporotischer Knochendefekte ist von eminenter klinischer Bedeutung, da nur auf diese Weise eine funktionell umfassende Rehabilitation möglich ist
Das als "goldener Standard" bezeichnete Verfahren der Entnahme autologen Knochens, meist aus dem Hüftkamm, zieht zusätzliche Kosten, Risiken und Belastungen des Patienten nach sich und es bestehen Grenzen hinsichtlich der zur Verfügung stehenden Knochenmenge. Die teilweise ausgedehnten Entnahmedefekte schmerzen oft noch sehr lange und es besteht ein erhöhtes Infektionsrisiko. Zur Vermeidung dieser Probleme wurden verschiedene alloplastische und allogene Materialien entwickelt, von denen bisher keines klinisch zufriedenstellende Ergebnisse zeigte ( Reuter, F., Kübler, N.R.: Die Wiederherstellung des Unterkiefers. Dtsch. Ärzteblatt 96 A, 1054ff. (1996)). Bisherige Verfahren der Defektauffüllung oder -regeneration (Bankmaterial, Kunststoffe, anorganische Werkstoffe) haben Nachteile und Risiken wie Virusinfektion, fibröse Umgebungsreaktion, Avitalität oder fehlende Resorption.
Die Entwicklung einer innovativen Gruppe von anorganischen Biomaterialien als Alternative zur autologen Osteoplastik stellt einen erheblichen Fortschritt dar, da eine Sekundäroperation mit ihren erhöhten Kosten, Risiken und Beschwerden vermieden werden kann und die Nachteile weiterer Verfahren, wie z.B. die Übertragung von Krankheiten (HIV, Hepatitis, Enzephalitis, u.a.) oder schwere Immunreaktionen auf das Implantat prinzipbedingt unterbleiben. Bei einer Verkürzung der Einheilphase bis zur Belastungsfähigkeit resultiert ein bedeutender Qualitätsgewinn für die Betroffenen.

Knochengewebsregeneration kann auf drei verschiedene Arten erfolgen: Osteogenese, Osteoinduktion sowie Osteokonduktion (Kübler, N.R.: Osteoinduktion und -reparation. Mund Kiefer Gesichts Chir. 1, 2ff. (1997)). Osteokonduktion bedeutet aus vorhandenem Knochengewebe entspringendes Wachstum entlang einer Leitstruktur, während man eine Stimulation der Differenzierung von Lagergewebszellen zu Osteoblasten als Osteoinduktion bezeichnet.

Osteogenese hingegen stellt eine Knochenneubildung aus vitalen, verpflanzten Knochenzellen dar.

Als wesentliche Forderung für ein Knochenersatzmaterial steht die Resorbierbarkeit. Knochen durchläuft kontinuierlich eine Phase eines Auf- und Abbaus, Remodeling genannt. Ein Knochenersatzmaterial soll nun an diesem Remodeling teilnehmen und damit in einer gewissen Zeit (je nach Defektgröße ca. 12 Monate) durch natürlichen Knochen ersetzt werden. Der Abbau des natürlichen Knochens erfolgt durch Osteoklasten. Bei einem idealen Knochenersatz soll die Resorption auch durch Osteoklasten erfolgen, da hiermit der Abbau des Materials an die Knochenneubildung gekoppelt ist. Alle anderen Resorptionsmechanismen laufen letztendlich über eine resorptive Entzündung, die - insbesondere wenn sie zu stark wird - immer einer Gewebeneubildung hemmt.

Knochen ist ein "Verbundmaterial" aus einem anorganischen mineralischen Anteil und einem organischen Anteil (Kollagen).
Das Mineral ist biogenes Hydroxylapatit (HA), ein Calziumphosphat. Reines HA hat die Strukturformel Ca₁₀(PO₄)₆(OH)₂. Biogenes HA weist dagegen einige Substitutionen auf. So finden sich Mg, F und Cl (< 1 wt %) gegen Ca substituiert und CO₃-Gruppen anstelle von PO₄- Gruppen (5,8 wt % in Knochen) (E.M. Carlisle: A possible factor in bone calcification, Science 167, pp.279-280(1970)). Die Kristallstruktur der Minerale ist hexagonal, wobei die Gitterparameter dem des synthetischen HA weitgehend entsprechen (Abweichungen in der 3. Dezimale, Angström-Bereich). Die zwischen den Kollagenfasern angeordneten Minerale weisen eine ausgeprägte Plättchenform auf. Die mittleren Abmessungen betragen 45 nm x 30 nm x 3 nm. Elektronenmikroskopische Untersuchungen belegen, dass es sich um Einkristalle mit Baufehlern handelt (E.M. Carlisle: In vivo requirement for silicon inarticular cartilage and connective tissue formation in the chick, J. Nutr. 106, pp.478-484(1976)), wahrscheinlich hervorgerufen durch die genannten Substitutionen. Die Mikrostruktur des Kollagen-Mineral-Verbundes lässt sich kurz wie folgt beschreiben. Kollagenfibrillen ordnen sich entsprechend der äußeren Belastung zu parallelen Bündeln. Diese werden durch zwischen den Fibrillen angeordnete HA-Kristalle mechanisch verstärkt. Die Plättchen liegen dabei flach auf den Fibrillen, wobei die kristallografische c-Achse der Minerale parallel zur Fibrillenlängsachse orientiert ist. Der Ort der Anlagerung an die Kollagenfasern ist durch die hierarchische Struktur des Kollagens (Molekül - Prokollagen (Tipelhelix) - Mikrofibrille) bestimmt. Prokollagenmoleküle lagern sich parallel mit einer charakteristischen Versetzung zusammen. In Längsrichtung findet man 35 nm-Lücken zwischen den Prokollagenmolekülen. Letztlich ergibt sich eine Struktur mit einer 64 nm-Periode (Parry, D.A.. The molecular and fibrillar structure of collagen and its relationship to the mechanical properties of connective tissue. Biophys Chem. 1988 Feb;29(1-2):195-209. Review). Aus dieser Basisstruktur bilden sich durch orientiertes Zusammenlagern der Fibrillen mehr oder weniger komplizierte Überstrukturen (Sehnen, Lamellenknochen, Geflechtknochen; Strukturmodelle siehe (Arsenault, A.L.: Crystal-collagen relationships in calcified turkey leg tendons visualized by selected-area dark field electron microscopy. Calcif Tissue Int. 1988 Oct;43(4):202-12.), (Traub, W.; Arad, T.; Weiner, S.: Origin of mineral crystal growth in collagen fibrils. Matrix. 1992 Aug;12(4):251-5.)und (Landis, W.J.; Hodgens, K.J.; Song, M.J.; Arena, J.; Kiyonaga, S.; Marko, M.; Owen, C., McEwen, B.F.: Mineralization of collagen may occur on fibril surfaces: evidence from conventional and high-voltage electron microscopy and three-dimensional imaging. J Struct Biol. 1996 Jul-Aug;117(1):24-35.)). Die Lücke zwischen den Prokollagenmolekülen wird als Ort der primären Keimbildung angesehen.

Für ein Knochenersatzmaterial ist es ideal, dass eine Porenstruktur, wie sie in der Spongiosa vorliegt, vorhanden ist. D.h. es müssen interkonnektierende Poren von ca. 0.2 mm bis 0.8 mm Durchmesser existieren. Dadurch ist es möglich, dass Blutgefäße in das Material einwachsen und somit der remodeling Prozess erst möglich wird.

Poröse Biokeramiken aus Tricalciumphosphat (TCP)/Hydroxylapatit (HA) und TCP/Monocalciumphosphat-monohydrat (MCPM) sind sowohl isoliert als auch in Kombination mit BMP sowie Knochenmarkzellen für Osteokonduktion und -induktion Gegenstand internationaler tierexperiementeller Forschungen (Wippermann, B. et al.: The influence of hydroxyapatite granules on the healing of a segmental defect filled with autologous bone marrow. Ann. Chir. Gynaecol. 88, 194ff (1999); Anselme, K. et al.: Associations of porous hydroxyapatite and bone marrow cells for bone regeneration. Bone 25 (Suppl. 2), 51Sff. (1999); Niedhart, C. et al.: BMP-2 in injizierbarem Tricalciumphosphat-carrier ist im Rattenmodell der autologen Spongiosaplastik biomechanisch überlegen. Z. Orthop. 137 (Suppl. I), VI-283 (1999); Penel, G. et al.: Raman microspectrometry studies of brushite cement: in vivo evolution in a sheep model. Bone 25(Suppl. 2), 81Sff. (1999);Brown, G.D. et al.: Hydroxyapatite cement implant for regeneration of periodontal osseous defects in humans. J Periodontol 69(2), 146ff (1998); Flautre, B. et al.: Volume effect on biological properties of a calcium phosphate hydraulic cement: experimental study in sheep. Bone 25 (Suppl. 2), 35Sff. (1999)).

Die offenporige gitterartige Struktur von resorbierbaren TCP/HA fördert die Regeneratbildung (Jansson, V. et al.: Knochen-/Knorpel-Regeneration in Bioimplantaten - Ergebnisse einer tierexperimentellen Studie. Z. Orthop. 137 (Suppl. I), VI-307 (1999)). Es gibt Hinweise, dass die Integration und Regeneration bei makroporösen HA-Keramiken durch Resorption, Mikrofraktur und erneute Osteokonduktion abläuft (Boyde, A. et al.: Osteoconduction in a large macroporous hydroxyapatite ceramic implant: evidence for a complementary integration and disintegration mechanism. Bone 24, 579ff. (1999)). Durch Kombination mit BMP (bone morphogenic protein (Meraw, S.J. et al.: Treatment of peri-implant defects with combination growth factor cement. J Periodontol 71(1), 8ff. (2000))) bzw. Osteoprogenitor-Zellen könnte eine weitere Steigerung des Regenerationspotentials durch zusätzliche Osteoinduktion erreicht werden.

Als Knochenersatz erweist sich ein Verbundmaterial aus organischen und anorganischen Materialien als ungünstig, da körperfremde organische Bestandteile Abstoßreaktionen des Körpers (Immunreaktionen) verursachen bzw. zu unerwünschten resorptiven Entzündungen führen.
In der Patentliteratur wird eine Vielzahl von porösen Keramiken als Knochenersatz beschrieben. In U.S. Patent 5,133,756A; 1992 wird die Keramik aus der Spongiosa von Rinderknochen hergestellt und hat damit die geforderte Porenstruktur. Die gesamte organische Matrix wird entfernt und der keramische Anteil wird bei Temperaturen von 1100 °C bis 1500 °C getempert.
Ein anderes Verfahren (U.S. Patent 4 861 733A; 1989) geht vom Gerüst natürlicher Korallen aus und wandelt das Kalziumcarbonat in einem hydrothermalen Prozess in Calciumphosphat um. Der Vorteil dieser Verfahren ist, dass die Porenstruktur (Größenverteilung, Morphologie) ideal zum Einwachsen des Knochengewebes ist.
Der entscheidende Nachteil dieser Keramiken ist, dass sie nicht resorbierbar sind. Für die beschriebenen Materialien bedeutet das, dass das Knochengewebe zwar ausgezeichnet in die Porenstruktur hinein wächst. Das feste Kristallgefüge der Keramik ist jedoch nicht am Knochenremodeling beteiligt. Es bleibt daher ein Fremdkörper und beeinflusst die mechanischen Eigenschaften. Insbesondere beim Knochenwachstum kommt es zu Entzündungen im Übergang vom Gewebe zur Keramik.

Resorbierbare Keramiken werden auf der Basis von Tricalciumphosphat beschrieben (US 5141511A, 1992 ). Auch hierbei handelt es sich um ein festes, durch Sinterprozesse entstandenes Kristallgefüge. Poren werden nur in der Größenordnung der Spongiosa in das Material eingebracht. Die Resorption erfolgt auf der Basis der Löslichkeit des Tricalciumphosphates. Damit tritt lokal eine erhöhte Ionenkonzentration auf und es kommt zu einer resorptiven Entzündung.

Bioaktive Gläser werden ebenfalls als Knochenersatzmaterial angeboten (US 6054400A, 2000; US 5658332A, 1997). Das anorganische Material liegt hier als glasiger Festkörper vor. Poren in der Größenordnung der Spongiosa erlauben ein Einwachsen des Gewebes. Kleinere Poren liegen in dem Material nicht vor.
Auch Glaskeramiken werden als Knochenersatz angeboten (US5981412A, 1999). Sie sind mit den bioaktiven Gläsern zu vergleichen, wobei das Calciumphosphat als kristalline Komponente in einer Glasmatrix vorliegt.

Als weitere Stoffgruppe für den Einsatz als Knochenersatz wurden Calciumphosphatzemente entwickelt (US5997624A, 1999; US5525148A, 1996). Entscheidender Nachteil dieser Stoffgruppe ist es, dass keine definierten interkonnektierenden Poren in das Material eingebracht werden, womit sie auf sehr kleine Knochendefekte beschränkt sind.

Das Patent US 5549 123 A, das z.B. ein Verfahren zur Erreichung einer bestimmten Porosität offenbart, enthält keine Beschreibung des Kristallgefüges, lediglich in Anspruch 8 wird ganz allgemein von Calciumphosphat gesprochen.
Beispielsweise zeigt DE 299 22 585 U1 einen Knochendefektfüller aus phasenreinem β-Tricalciumphosphat mit zwei Porensystemen. Das Kristallgefüge als entscheidendes charakteristisches Material wird nicht näher beschrieben. β-Tricalciumphosphat hat gegenüber Hydroxylapat den Nachteil, dass durch die Löslichkeit eine erhöhte Ca-Ionenkonzentration auftritt und damit die Gewebeneubildung durch eine resorptive Entzündung gehemmt wird.
Das Patent GB 2348 872 A betrifft einen porösen Calciumphosphat-Körper mit Poren von 50 µm an aufwärts, der das charakteristische Kristallgefüge der später beschriebenen Erfindung nicht aufweist. Damit werden auch die wesentlichen neuen Eigenschaften des erfindungsgemäßen Materials nicht erreicht.
Ferner ist auf das Patent DE 198 25419 A zu verweisen, wonach es bekannt ist, eine resorbierbare Calciumphosphatkeramik mit Poren im Nanometerbereich sowie mit Poren im Größenbereich von einigen hundert µm bis in den mm-Bereich herzustellen.

Bekannt ist schließlich durch die DE 197 21 661 A ein Verfahren zur Herstellung eines resorbierbaren Knochenersatzmaterials mittels ,jet phase solidification", ein Verfahren, bei dem mit einer feinen Spritzdüse der Werkstoff Spur um Spur und Schicht für Schicht aufgetragen wird.
Der vorliegenden Erfindung liegt demgegenüber die Aufgabe zugrunde, ein Verfahren zur Herstellung eines Knochenersatzwerkstoffes zu liefern, der eine Bildung von Knochengewebe unterstützt ( der also osteokonduktiv bzw. osteoinduktiv ist) und der über die natürlichen Prozesse des Knochenremodeling resorbiert wird.
Diese Aufgabe wird erfindungsgemäß durch ein Verfahren mit den Merkmalen des Anspruches 1 und ein nach diesem Verfahren erhältliches Material gelöst. Das erfindungsgemäße Verfahren besteht darin, dass
- einem SiO₂-Sol kristallines Calciumphosphat-Pulver zugemischt wird,
- vor dem abgeschlossenen Gelübergang die sich bildende hochviskose Suspension durch eine Düse oder ein Düsensystem mit einem Durchmesser der Düsen zwischen 50 µm bis 1000 µm gedrückt wird, wobei die Viskosität der Suspension derart ist, dass die sich bildenden Fäden nicht ineinander verlaufen,
- die aus der Düse austretenden Fäden derart in eine Form gepackt werden, dass die Fäden sich an den Berührungsstellen verbinden und dass ein Volumenanteil von bevorzugt 50 % der durch die Packung der Fäden determinierten Poren erreicht wird,
- das Material nach dem Altem der Gelstruktur vorzugsweise in einem Temperaturbereich von 90°C bis 200°C getrocknet wird.

Zur Herstellung der hochviskosen Suspension wird Calciumphosphatpulver oder -granulat, das durch die verwendete Komponente, die Korngrößenverteilung, die Morphologie, den Kristallinitätsgrad und vorhandene Gitterdefekten variierbar ist, mit einem SiO₂-Sol möglichst homogen vermischt.
Anschließend wird das Gemisch in einen Behälter so gefüllt, dass sich keine Luft in dem geschlossenen Behälter befindet und der Behälter wird um eine horizontale Achse gedreht, um ein Absinken der schwereren Feststoffanteile zu verhindern.
Der Durchmesser der Düse bzw. der Düsen liegt vorzugsweise im Bereich von 50 µm bis 1000 µm, wobei mit 200 µm ein Wert erreicht wird, der dem Durchmesser der Trabekel im Knochen entspricht und technisch gut zu realisieren ist.
Die durch die Düsen oder das Düsensystem entstehenden Fäden aus der hochviskosen Suspension werden derart in die beliebige Form, wie Zylinder, Hohlzylinder oder Kugelschalensegment gedrückt, dass die durch die Packung der Fäden determinierten Poren einen bestimmten Volumenanteil von bevorzugt 50% erhalten und eine Verbindung der sich berührenden Fäden gewährleistet ist.
Die Viskosität der die Fäden bildenden Suspension darf nicht so niedrig sein, daß die Fäden ineinander verlaufen.
Gegebenenfalls, insbesondere wenn sehr dünne Fäden hergestellt werden sollen, kann es notwendig sein, die Viskosität nach Passieren der Düse bzw. der Düsen zu erhöhen, um zum einen ein Verstopfen der Düsen zu verhindern (geringere Viskosität) und zum anderen ein Verlaufen der Fäden zu vermeiden (höhere Viskosität).
Das wird dadurch erreicht, dass der Suspension nach Verlassen der Düse(n) schnell Lösungsmittel entzogen wird. Das kann durch eine schnelle Temperaturerhöhung und bzw. oder durch eine Verringerung des Partialdruckes des Lösungsmittels erfolgen. Am einfachsten erweist es sich, die Fadenpackung mit heißer trockner Luft zu spülen.
Um die Festigkeit des hochporösen Formkörpers zu verbessern, kann die Packung der Fäden mit einer Suspension gleicher Zusammensetzung wie die Ausgangssuspension getränkt werden, wobei hierbei eine Viskosität der Suspension gewählt wird, die gewährleistet, dass Teile der Suspension zwischen den Fäden hängen bleiben und mit der Gelbildung eine bessere Verknüpfung der Fäden ermöglicht und gleichzeitig ein Verstopfen der großen interkonnektierenden Poren verhindert wird.

Vor dem Trocknen des Formkörpers erfolgt ein Altern der Gelstruktur. Eine gesättigte Lösungsmittelatmosphäre verhindert das vorzeitige Trocknen.
Das Trocknen wird anschließend bei einer Temperatur von bevorzugt 90-150°C 2 Stunden lang durchgeführt. Das Gel bleibt dann in einem nanoporösem Zustand, was die Resorption erleichtert. Soll die Festigkeit erhöht werden erfolgt eine Temperaturbehandlung in einem Bereich zwischen 600°C und 800°C.
Nach dem Trocknen erfolgt ein Puffern des hochporösen Formkörpers bevorzugt mit Phosphatpuffer bei pH 7,2.
Der danach erforderliche Trocknungsprozess wird mit einer Sterilisation verbunden.

Das durch das erfindungsgemäße Verfahren erhaltene Material besitzt ein lockeres Kristallgefüge von Calciumphosphaten, d.h. die Kristallite sind nicht wie in einem Festkörper (Keramik) dicht zusammengefügt, sondern nur über einige Molekülgruppen miteinander verbunden. Das Volumen, das im natürlichen Knochen vom Kollagen eingenommen wird, ist in dem Material als interkonnektierende Poren im Nanometerbereich vorhanden. Eine zweite Porengröße, ebenfalls interkonnektierend und im Bereich von einigen Mikrometern, ermöglicht ein Einwachsen von Kollagenfasern bei der Gewebebildung. Diese Fasern sind Keimbildner für die einsetzende Biomineralizierung ( Bildung des körpereigenen biologischen Apatits). Das Material enthält eine dritte interkonnektiernde Porenkategorie, die der Spongiosa nachempfunden ist und damit im Bereich von ca. 100µm bis 1000µm liegt und damit ein Einwachsen von Blutgefäßen ermöglicht, wodurch die Resorption und die Knochenneubildung nicht nur als Front vom gesunden Knochen aus erfolgt, sondern aus dem gesamten Defekt heraus geschieht.

Durch die Porenstruktur ist das entwickelte Material hervorragend geeignet, körpereigene (z.B. Knochenmarksflüssigkeit) oder körperfremde (z.B. BMPs) osteoinduktive Komponenten aufzunehmen. Hierdurch wird eine extreme Gewebefreundlichkeit und damit ein schnelles Einwachsen von Knochengewebe erreicht.
Das lockere Kristallgefüge macht eine Resorption durch Osteoklasten möglich.
Als Calciumphospat wird vorrangig ein Hydroxylapatit verwendet, das in der Kristallitgröße dem biologischen Apatit angepasst ist. Eine zweite lösliche Calciumphosphatkomponente (β-Tricalciumphosphat oder Bruschit) kann als lokaler Calciumphosphatliferant für die an den Kollagenfasern beginnende Biomineralisierung gewählt werden. Die löslichen Komponenten sollen in der Konzentration vorliegen, dass keine bzw. nur eine geringe resorptive Entzündung auftritt, die die Gewebeneubildung nicht verhindern soll.

In der Literatur wird zunehmend vom positiven Einfluss von SiO₂ auf die Kollagen- und Knochenbildung berichtet.
Die Ergebnisse wurden sowohl bei in vitro als auch bei in vivo Experimenten erhalten.
Calisle (E.M. Carlisle: A possible factor in bone calcification, Science 167, pp.279-280(1970)) berichtet, dass Silizium ein wichtiges Spurenelement bei der Bildung und Mineralisation der Knochen ist. Siliziummangel erzeugt bei Hühnern und Ratten im Tierexperiment einen defekten Knochenaufbau (E.M. Carlisle: In vivo requirement for silicon inarticular cartilage and connective tissue formation in the chick, J. Nutr. 106, pp.478-484(1976)). Das Silizium wird von verschiedenen Autoren in unterschiedlichen Formen bei den Experimenten verwendet. So nutzen Keeting et al. ( P.E. Keeting et al.: Zeolite a increases proliferation, differentation, and transforming growth factor β production in normal adult human osteoblast-like cells in vitro, J. of bone and mineral research, Vol.7, Nr.11, pp. 1281-1289(1992)) siliziumhaltige Zeolite A für ihre Experimente und stellen einen positiven Einfluss auf das Zellwachstum und die Zellteilung von kultivierten Zellen einer humanen Zelllinie fest. Hierbei ist natürlich wichtig, dass damit auch andere Elemente, wie z.B. Aluminium, mit einer negativen Wirkung in das System gelangen.
Der Einfluss von Silizium auf die Knochenbildung wird von Reffitt et al. (D. Reffitt et al.: Silicon stimulated collagen type I synthesis in human osteoblast-like cells, Bone 23(5), p.419(1998) ) in vitro an Zelllinien untersucht. Es wird eine Stimulierung der Kollagen Typ I Synthese festgestellt.
Im Tierexperiment wurde der Knochenmasseverlust von osteoporotischen Ratten untersucht (H. Rico et al.: Effect of silicon supplement on osteopenia induced by ovariectomy in rats, Calcif. Tissue Int. 66(1), pp. 53ff(2000) ). Hierbei wurde festgestellt, dass Ratten, die 500mg Si pro kg Nahrung erhielten, keinen Knochenmasseverlust zeigten im Gegensatz zu den Tieren, die kein Si in der Nahrung hatten. Lyu (K. Lyu, D.Nathason, L. Chou: Induced osteogenesity In vitro upon composition and concentration of silicon, calcium, and phosphorous. Sixth World Biomaterials Congress Transactions 2000, 1387) stellt mit in vitro Experimenten fest, dass Si eine bedeutende Rolle bei der Osteogenese spielt und eine Korrelation zwischen Osteogeneseaktivität und Si-Konzentration (von 10 bis 100ppm Si im Kulturmedium) besteht.

Der positive Aspekt des SiO₂ bei der Knochenbildung wird durch das beschriebene Knochenersatzmaterial aufgegriffen, indem in das lockere Kristallgefüge des Knochenersatzmaterials nanoporöses SiO₂ eingefügt wird. Es wird nanoporöses SiO₂ gewählt, um zum einen eine gute Löslichkeit zu realisieren und zum anderen eine hohe innere Oberfläche zu gewährleisten.

Die Erfindung wird im folgenden an Hand von Beispielen erläutert. Sie ist jedoch auf diese Beispiele nicht beschränkt.

### Beispiel 1

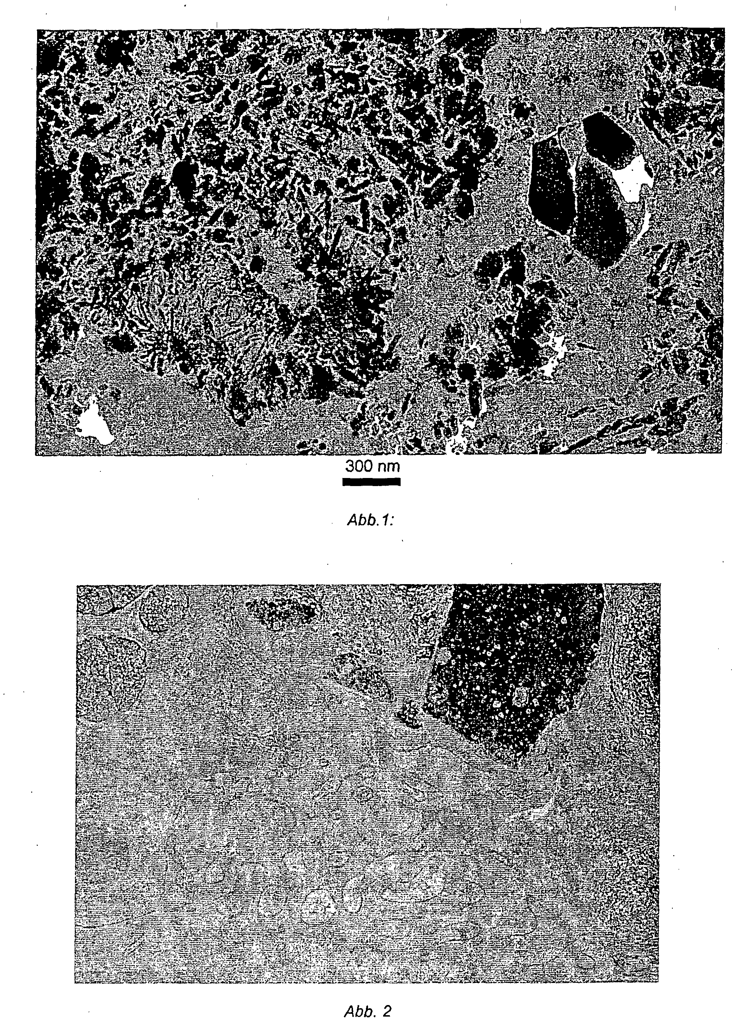

Die Abbildung. 1 zeigt eine transmissionelektronenmikroskopische Aufnahmen von Schnitten des in Epoxid eingebetteten Biomaterials. Die glatten Flächen sind die mit Epoxid gefüllten Poren. Deutlich zu erkennen ist das lockere Kristallgefüge, das durch unterschiedliche Calciumphosphatpulver mit unterschiedlicher Kristallmorphologie beeinflusst werden kann. Für dieses Beispiel wurde beim Calciumphosphat ein Verhältnis 60% Hydroxilapatit (HA) und 40% β Tricalciumphosphat (TCP) gewählt. Die größeren Kristallite in der Abbildung sind die löslichen β TCP Anteile.

Die Porosität hat die Größenordnung der Kristallite. So existiert eine große Oberfläche, die in vivo von Körperflüssigkeit benetzt wird.
Die Abbildung demonstriert gleichzeitig, dass im µm-Bereich ausgeprägte interkonnektierende Poren existieren (hier durch die TEM-Präparation mit Epoxid gefüllt), die ein ungehindertes Einwachsen von Kollagenfasern erlauben.
Göttinger Minischweine wurden für die Tierexperimente genutzt. Die Tiere waren adult (ein Jahr alt) und hatten ein Gewicht zwischen 25 und 30 kg. Die Knochendefekte überschritten die kritische Größe von 5 cm³; ihre Abmessungen betragen ca. 3,0cm*1,5cm*1,5 cm. Sie wurden in den Unterkiefer gesetzt, komplett mit dem Knochenersatzmaterial gefiillt und mit Periost geschlossen. Nach 5 Wochen wurden die Schweine getötet, und die Unterkiefer entnommen und röntgenologische, histologische und rastermikroskopische Untersuchungen durchgeführt. Die Tierversuche wurden nach 5 Wochen ausgewertet, um das Anfangsstadium der Knochenregeneration zu studieren.
Im Randbereich ist eine gute Verknöcherung nachzuweisen. Histologische Schnitte aus dem Randbereich dokumentieren eine sehr gute Knochenbildung Das Biomaterial wird von jungen Knochen z.T. ummantelt (Abb.2).

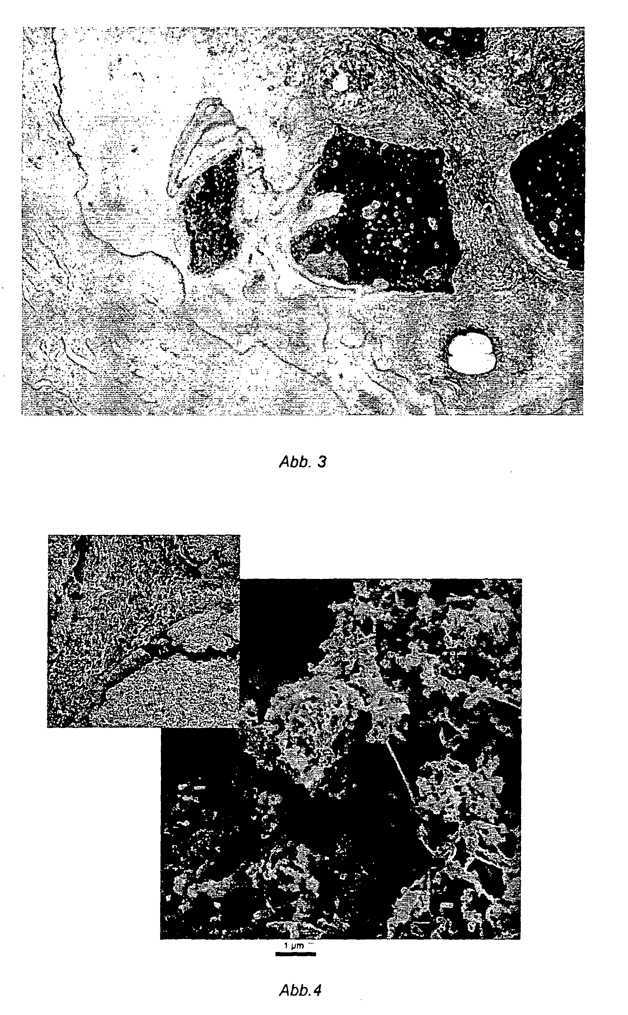

Es sind schon nach 5 Wochen deutliche Anzeichen der Resorption zu erkennen. Das ursprünglich "runde" Material hat Kanten und Ecken bekommen und zeigt Einbuchtungen, wie sie typisch für Osteoklastenaktivitäten sind (Abb. 3). Zudem ist zu erkennen, dass die Mikrometerporen des Materials von organischem Material durchsetzt sind. Die REM-Aufnahmen bestätigen das eindrucksvoll. In Abbildung 4 ist eine rasterelektronenmikroskopische Aufnahme eines Schnittes aus der Mitte des Defekts und eine Ausschnittsvergrößerung dargestellt. Im gesamten Defekt - auch zentral, wo die Knochenbildung noch nicht soweit vorangeschritten ist - sind die Mikroporen von Kollagenfasern durchzogen, die wiederum deutlich eine Mineralisierung zeigen.

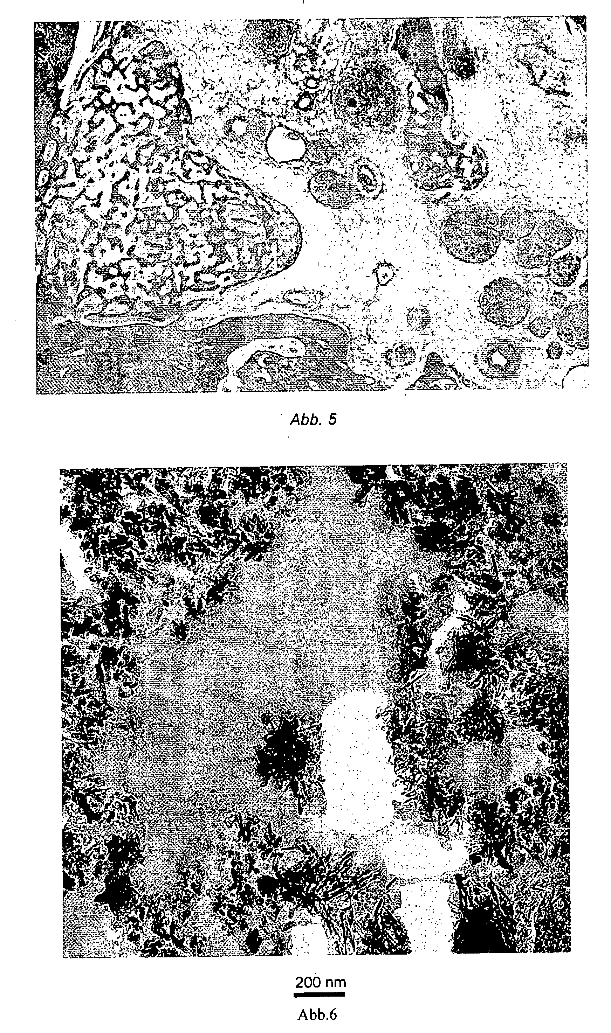

Die Abbildung 5 zeigt einen demineralisierten histologischen Schnitt (Hämalaun Eosin). Es ist zu erkennen, dass die großen Poren des Biomaterials ein vom Rand beginnendes Einwachsen von Blutgefäßen erlauben.

### Beispiel 2

Die Abbildung 6 zeigt eine transmissionelektronenmikroskopische Aufnahmen von Schnitten des in Epoxid eingebetteten Biomaterials. Die glatten Flächen sind wiederum die mit Epoxid gefüllten Poren. Deutlich zu erkennen ist das lockere Kristallgefüge, das sich von dem in Abbildung 1 unterscheidet. Für dieses Beispiel wurde als Calciumphosphat reines Hydroxylapatit (HA) verwendet.
Die Porosität hat die Größenordnung der Kristallite. So existiert eine große Oberfläche, die in vivo von Körperflüssigkeit benetzt wird.
Die Abbildung demonstriert gleichzeitig, dass im µm-Bereich ausgeprägte interkonnektierende Poren existieren (hier durch die TEM-Präparation mit Epoxid gefüllt), die ein ungehindertes Einwachsen von Kollagenfasern erlauben.

### Beispiel 3

60 ml Tetraethoxysilan werden unter Rühren 18 ml Wasser und 18 ml Salzsäure Masslösung zugegeben.
Nach der Hydrolyse wird diesem Ansatz ca. 60 g Hydroxylapatit und 40g β Tricalciumphosphat zugegeben. Diese Suspension wird in einem geschlossenen zu 100% gefüllten Gefäß um eine horizontale Achse gedreht, um eine Ablagerung der Phosphate am Boden zu verhindern.
Nach 2 Stunden ist die Viskosität so hoch, dass das Sol durch eine Düse mit 1mm Durchmesser gedrückt wird und stabile Fäden erzeugt, die als zufällige Packung in eine recheckige Form gebracht werden, dass die Fäden ca. 50% Raumausfüllung ergeben.
Die Probe wird nun 12 h im Exikator bei gesättigtem Ethanoldampf gelagert. Anschließend erfolgt eine Trocknung bei 120 °C 2 h in einem Ofen.

Nach dem Trocknungsprozeß wird mittels Phosphatpuffer ein pH-Wert von 7.2 eingestellt.
Die Proben werden an der Luft getrocknet, später bei 200°C (Aufheizrate: 1°C/min; Dauer: 3 Stunden) getrocknet und sterilisiert.

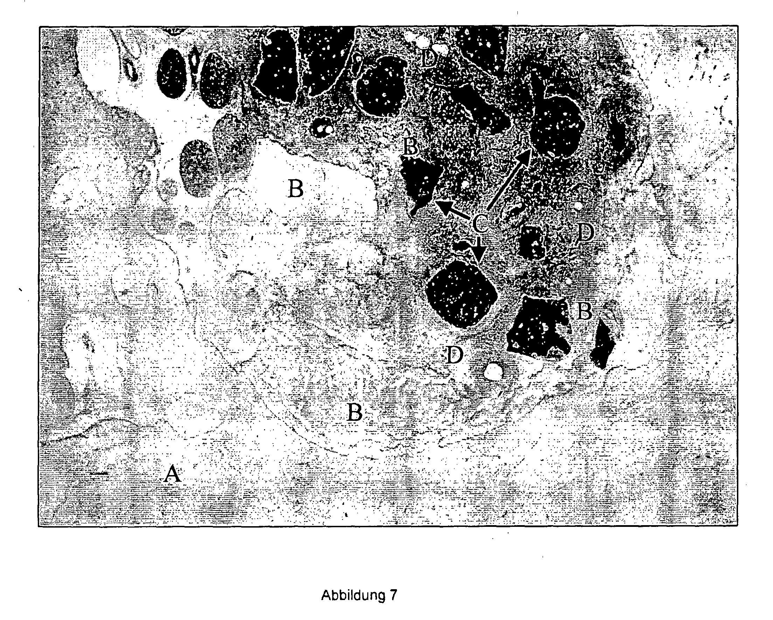

Die Tierexperimente wurden mit Göttinger Minischweinen (ausgewachsen, ca. 60 kg schwer) durchgeführt. Dabei wurde ein ca. 5 cm³ großer Defekt im Unterkiefer gesetzt und mit dem Material gefüllt. Nach fünf Wochen wurden die Schweine getötet, um das Anfangsstadium der Regeneration des Defekts auszuwerten. Eine lichtmikroskopische Abbildung eines histologischen Schnitts ist in Abbildung 7 dargestellt. Es ist ein extrem schnelles Wachstum des Knochens (einschließlich von Blutgefäßen) in den Poren des Knochenersatzmaterials und eine Resorption des Materials zu erkennen. (A- Knochen des Unterkiefers; B - neugebildeter Knochen; C - Reste des Knochenersatzmaterials; D - Blutgefäße in den Poren des Materials). Die ursprünglich fadenförmige Struktur hat sich durch Resorption stark verändert.

### Beispiel 4

60 ml Tetraethoxysilan werden unter Rühren 18 ml Wasser und 18 ml Salzsäure Maßlösung zugegeben.
Nach der Hydrolyse wird diesem Ansatz ca. 40 g Hydroxylapatit zugegeben. Diese Suspension wird in einem geschlossenen zu 100% gefüllten Gefäß um eine horizontale Achse gedreht, um eine Ablagerung der Phosphate am Boden zu verhindern.
Nach 2 Stunden ist die Viskosität so hoch, dass das Sol durch eine Düse mit 0.2mm Durchmesser gedrückt wird und stabile Fäden erzeugt, die als zufällige Packung in eine rechteckige Form gebracht werden, dass die Fäden ca. 50% Raumausfüllung ergeben.
Die Probe wird nun 12 h im Exikator bei gesättigtem Ethanoldampf gelagert. Anschließend erfolgt eine Trocknung bei 120 °C 2 h in einem Ofen.

## Patentansprüche

1. Verfahren zur Herstellung eines anorganischen, resorbierbaren Knochenersatz-materials, bei dem eine Calciumphosphatsuspension aus einer Düse gedrückt wird und das aus den Fäden sich bildende Material eine offene Porenstruktur aufweist, **dadurch gekennzeichnet, dass**
• einem SiO₂-Sol kristallines Calciumphosphat-Pulver zugemischt wird,
• vor dem abgeschlossenen Gelübergang die sich bildende hochviskose Suspension durch eine Düse oder ein Düsensystem mit einem Durchmesser zwischen 50 µm bis 1000 µm gedrückt wird, wobei die Viskosität der Suspension derart ist, dass die sich bildenden Fäden nicht ineinander verlaufen,
• die aus der Düse austretenden Fäden derart in eine Form gepackt werden, dass die Fäden sich an den Berührungsstellen verbinden und dass ein Volumenanteil von bevorzugt 50 % der durch die Packung der Fäden determinierten Poren erreicht wird,
• das Material nach dem Altern der Gelstruktur vorzugsweise in einem Temperaturbereich von 90° C bis 200° C getrocknet wird.

2. Verfahren nach Anspruch 1, **dadurch gekennzeichnet, dass** die Viskosität der Suspension nach dem Verlassen der Düse oder des Düsensystems zusätzlich vergrößert wird, indem durch eine schnelle Erhöhung der Temperatur der Fäden Lösungsmittel verdampft.

3. Verfahren nach Anspruch 1, **dadurch gekennzeichnet, dass** die Viskosität der Suspension nach dem Verlassen der Düse oder des Düsensystems zusätzlich vergrößert wird, indem durch eine schnelle Verringerung des Partialdruckes des Lösungsmittels der Suspension Lösungsmittel verdampft.

4. Verfahren nach Anspruch 1, **dadurch gekennzeichnet, dass** die Packung der Fäden mit einer Suspension gleicher Zusammensetzung wie die Ausgangssuspension getränkt wird, wobei hierbei eine Viskosität der Suspension gewählt wird, die gewährleistet, dass Teile der Suspension zwischen den Fäden hängen bleiben und mit der Gelbildung eine bessere Verknüpfung der Fäden ermöglicht wird.

5. Anorganisches resorbierbares Knochenersatzmaterial, erhältlich durch ein Verfahren nach Anspruch 1.

## Claims

1. Method for producing an inorganic, resorbable bone substitute material, in which a calcium phosphate suspension is forced out of a nozzle and the material forming from the filaments has an open pore structure, **characterized in that**
• crystalline calcium phosphate powder is admixed with a SiO₂ sol,
• before the completed gel transformation, the highly viscous suspension forming is forced through a nozzle or nozzle system with a diameter of between 50 µm and 1000 µm, the viscosity of the suspension being such that the filaments forming do not run into one another,
• the filaments emerging from the nozzle are packed into a mould in such a way that the filaments are interconnected at the points of contact and **in that** a proportion by volume of preferably 50% of the pores determined by the packing of the filaments is achieved,
• after ageing of the gel structure, the material is dried, preferably in a temperature range of from 90°C to 200°C.

2. Method according to Claim 1, **characterized in that** the viscosity of the suspension after leaving the nozzle or the nozzle system is additionally increased by evaporating solvent by rapidly increasing the temperature of the filaments.

3. Method according to Claim 1, **characterized in that** the viscosity of the suspension after leaving the nozzle or the nozzle system is additionally increased by evaporating solvent by rapidly reducing the partial pressure of the solvent of the suspension.

4. Method according to Claim 1, **characterized in that** the packing of the filaments is impregnated with a suspension of the same composition as the initial suspension, choosing for this a viscosity of the suspension which ensures that parts of the suspension remain suspended between the filaments, and better interlinkage of the filaments being made possible with the gel formation.

5. Inorganic resorbable bone substitute material, obtainable by a method according to Claim 1.

## Revendications

1. Procédé de fabrication d'un matériau inorganique résorbable de remplacement d'os, lors duquel une suspension de phosphate de calcium est pressée à travers une buse et lors duquel le matériau se formant à partir des fils présente une structure de pores ouverte, **caractérisé**
• en ce qu'une poudre de phosphate de calcium cristalline est mélangée à un sol de SiO₂,
• en ce que, avant la transition achevée en gel, la suspension hautement visqueuse qui se forme est pressée à travers une buse ou un système de buses, ayant un diamètre compris entre 50 µm et 1000 µm, la viscosité de la suspension étant telle que les fils qui se forment ne coulent pas les uns dans les autres,
• en ce que les fils émergeant de la buse sont tassés en une forme telle que les fils se lient aux points de contact et qu'une proportion volumique, de préférence, de 50 %, des pores déterminées par le tassement des fils est obtenue,
• en ce que le matériau, après vieillissement de la structure de gel, est séché, de préférence, dans un domaine de température de 90°C à 200°C.

2. Procédé selon la revendication 1, **caractérisé en ce que** la viscosité de la suspension après avoir quitté la buse ou le système de buses, est encore augmentée par l'intermédiaire d'une évaporation du solvant, grâce à une augmentation rapide de la température des fils.

3. Procédé selon la revendication 1, **caractérisé en ce que** la viscosité de la suspension après avoir quitté la buse ou le système de buses, est encore augmentée par l'intermédiaire de l'évaporation du solvant, grâce à une diminution rapide de la pression partielle du solvant de la suspension.

4. Procédé selon la revendication 1, **caractérisé en ce que** l'ensemble tassé de fils est imprégné à l'aide d'une suspension de composition identique à celle de la suspension de départ, une viscosité de la suspension étant choisie ici qui garantisse que des parties de la suspension restent accrochées entre les fils et qu'une meilleure connexion des fils soit rendue possible par la formation de gel.

5. Matériau inorganique résorbable de remplacement d'os, que l'on peut obtenir grâce à un procédé selon la revendication 1.
